# EUROPEAN PATENT SPECIFICATION

(11) **EP 3 888 634 B1**
(45) Date of publication and mention of the grant of the patent: **07.06.2023**
(21) Application number: 20167272.2
(22) Date of filing: 31.03.2020
(51) Int. Cl.: A61K 9/107, A61K 47/26, A61K 47/36, A61K 47/44, A61K 36/47, A61K 31/728, A61P 27/04

(54) **OPHTHALMIC FORMULATION AND ITS USE**
OPHTHALMISCHE FORMULIERUNG UND DEREN VERWENDUNG
FORMULATION OPHTALMIQUE ET SON UTILISATION

(43) Date of publication of application: 06.10.2021
(73) Proprietor: Oy Finnsusp Ab, 21420 Lieto (FI)
(72) Inventor: Laihia, Jarmo, 21420 Lieto (FI)
(74) Representative: Berggren Oy

(56) References cited:
- EP-A1- 0 480 690
- WO-A1-2019/036625
- JARMO LAIHIA ET AL: "Disease aetiology-based design of multifunctional microemulsion eye drops for moderate or severe dry eye: a randomized, quadruple-masked and active-controlled clinical trial", ACTA OPHTHALMOLOGICA: THE OPHTHALMOLOGICAL JOURNAL OF THE NORDIC COUNTRIES, vol. 98, no. 3, 3 October 2019 (2019-10-03), pages 244-254, XP055724632, Denmark ISSN: 1755-375X, DOI: 10.1111/aos.14252
- Clinical Study Protocol - Piiloset Trehalose Emulsion Eye Drop; pages 1-4 and 16-18

## Description

The present invention relates to an ophthalmic formulation and its use according to the preambles of the enclosed independent claims.

The tear film has an invaluable role for ocular health in protecting and moisturizing the corneal and conjunctival surfaces of the eye. It also forms a refracting surface for light entering the visual system. Tear film is the part of tear fluid that spreads onto the ocular surface when the eyelids are opened, thus called the preocular tear film. The structure of the preocular tear film has been traditionally described using a three-layer model: the innermost mucin layer covers and attaches to the surface of the ocular epithelium; the aqueous layer provides lubrication, nutrients, antimicrobial proteins and appropriate osmolarity to maintain the viability of the exposed ocular cells; and the outermost lipid layer prevents excessive evaporation of water from the aqueous layer. The aqueous and mucin layers have been later commonly considered to form a single mucoaqueous layer instead of two distinct layers.

The tear film lipid layer is derived from excretions of the meibomian glands at the lid margins and is spread onto the tear film with each blink. Certain physical and rheological characteristics, such as surface tension, viscosity, pH and osmolarity, dictate the ability of the tear film to establish a stabile protective and moisturizing layer onto the ocular surface after spreading. Defects in these key characteristics may lead to impairment of tear film function, which may manifest as ocular surface disorders including, but not limited to, dry eye disease, blepharitis, Stevens-Johnson syndrome, Sjögren's syndrome, ocular cicatricial pemphigoid and graft-versus-host disease.

Dry eye is the most common ocular surface disorder and these terms are often even used interchangeably. Dry eye can be caused by multiple intrinsic, environmental or iatrogenic factors. It is known that hyperosmolarity and elevated surface tension are found in tears of patients with dry eye, leading to desiccation-induced stress and death of ocular epithelial cells. The physiological surface tension in the tears of healthy individuals is reported to be in the range 38-46 mN/m. In tears from patients with dry eye, the values have increased close to 50 mN/m; simultaneously, the spreading of the tear film is remarkably retarded. Elevation of both osmolarity and surface tension may result from a defective tear film lipid layer. In addition, instability of the preocular tear film, observable as a short tear film break-up time representing the retention time of tear film on the surface of the open eye, is a consequence of poor quality and slow spreading of the tear film during blink cycles. These disturbances in the tear film lead to epithelial damage, sensations of uncomfortable or painful ocular symptoms and impaired visual function.

Typical first-line treatment options for dry eye include topically administered ocular lubricants such as eye drops, gels and sprays. The ultimate aim of dry eye management is to restore homeostasis of the ocular surface and to prevent a return to the vicious cycle of dry eye. Lipid-containing eye drops have the advantage to support the tear film lipid layer. They can often be formulated as various types of dispersions, such as emulsions. Preferably the emulsions used as ocular lubricants should be optically transparent, stable and homogenous, to avoid blurring of vision and requiring no shaking or inverting of the dispensing bottle to restore homogeneity of the emulsion prior to application to the eye.

Laihia et al. (Acta Ophthalmol. 2019 Oct 3. doi: 10.1111/aos.14252) discloses the results of a randomized clinical trial in which patients with dry eye were treated with microemulsion eye drops (a non-commercial investigational product) comprising 0.1% sacha inchi (*Plukenetia volubilis*) seed oil. The ophthalmic formulation was optically clear and stable. The authors suggest that the observed improvement in ocular surface protection by the tear film resulted from strengthening of the tear film lipid layer and lowering of its surface tension. However, there is no indication about the surface tension of the formulation used.

The object of the present invention is to minimize or even eliminate the disadvantages existing in the prior art.

An object of the present invention is to provide an ophthalmic formulation that is optically transparent, thermodynamically stable and with a physiological surface tension which corresponds to healthy human tears.

A further object of the present invention is to provide an ophthalmic formulation that is suitable for treatment or prevention of ocular surface disorders, especially exemplified by impaired tear film stability, retention or spreading, or by tear film hyperosmolarity.

All the described embodiments and advantages apply to all aspects of the present invention, both the formulation and its uses, whenever applicable, even if not always explicitly stated so.

These objects are achieved by the features disclosed in the independent claims and the invention is defined by the features of the enclosed independent claims. Some preferable embodiments of the present invention are presented in the dependent claims.

The ophthalmic formulation in form of an oil-in-water microemulsion according to the present invention comprises oil particles dispersed in a continuous water phase, the formulation comprising
- 0.1 - 2.0 weight-%, of a natural oil which is sacha inchi seed oil, and
- at least a first non-ionic emulsifier, which is polyoxyethylene sorbitan mono-oleate, and a second non-ionic emulsifier, which is sorbitan mono-oleate, wherein the first non-ionic emulsifier and the second non-ionic emulsifier have a total molar concentration which is 80 -150 % of the total molar concentration of unsaturated fatty acid(s) of the natural oil,
wherein the formulation has a surface tension in a range of 40 - 43 mN/m at an emulsion-air interface at a temperature of 15 - 30 °C.

Typical formulation according to the present invention is for use as a medicament.

Typical formulation according to the present invention is for use in a treatment of an ocular surface disorder.

Now it has been surprisingly found that an ophthalmic formulation comprising oil-in-water microemulsion according to the present invention has a surface tension adjusted in the range of 40 - 43 mN/m. It is assumed, without wishing to be bound by a theory, that within this surface tension range the ophthalmic formulation is capable of effectively counteracting the adversely elevated surface tension of tear fluid in patients with an ocular surface disease, such as dry eye. The surface tension in the range of 40 - 43 mN/m represents a specific selected sub-range within the physiological range 38 - 46 mN/m present in the tear fluid of healthy non-dry-eye individuals. The ophthalmic formulation according to the invention unexpectedly enhances the retention of the tear film and the protecting effect of the tear film on the ocular surface. Furthermore, the ophthalmic formulation according to the invention may decrease, to a clinically meaningful extent, the tear film hyperosmolarity, and the damage and inflammation on the ocular epithelium, all of which are currently acknowledged core etiologic factors of the vicious cycle of dry eye. By correcting, with the aid of the inventive formulation, the surface tension of the tear film of dry eye patients, the spreading of the tear film is enhanced, which improves the physiological protecting function of the preocular tear film. These advantageous effects together result in the recovery of a healthy ocular surface epithelium and tear film function, with reduced symptoms.

The formulation according to the invention has a surface tension in a range of 40 - 43 mN/m, or 40.5 - 43 mN/m, at the emulsion-air interface at a temperature of 15 - 30 °C. The surface tension of a solution may be measured by a number of techniques known in the art, including force tensiometer or du Noüy ring method, pendant drop or sessile drop methods, Wilhelmy plate tensiometer method or bubble pressure tensiometer method. The specific surface tension of the formulation of the present invention is not only advantageous for retaining and/or stabilising the tear film of the eye. The surface tension also affects the volume of emulsion drops when administered on the eye. The specific surface tension range of the formulation according to the present invention can surprisingly reduce the volume of the drops administered from a standard eye drop bottle to a desirable drop volume of about 30 µl, which corresponds to the maximal volume of liquid the conjunctival sac of the eye is able to hold.

In the present context "oil-in-water microemulsion" denotes an emulsion comprising discrete oil particles dispersed in a continuous water phase. The oil particles are discrete particles or droplets which are uniformly dispersed in the continuous water phase. The formulations can be prepared by self-emulsification techniques, known as such for a person skilled in the art, avoiding excessive energy demands and laborious processing steps in the manufacture.

It has been observed that the sacha inchi seed oil has an unsaturated fatty acid content that has, among others, a beneficial effect on the recovery of a healthy ocular surface epithelium. Natural oils may further comprise residual amounts of diglycerides, monoglycerides, free fatty acids, plant sterols, pigments, glucosides, and natural antioxidants, such as vitamins.

The formulation according to the present invention comprises at least a first non-ionic emulsifier and a second non-ionic emulsifier, which are different from each other. The first non-ionic emulsifier and the second non-ionic emulsifier are structurally compatible with the unsaturated fatty acid chains of the natural oil triglycerides present in the formulation. In this manner it is possible to enhance the emulsifying efficiency of the emulsifiers and to produce the desired surface tension and oil particle size for the ophthalmic microemulsion formulation.

The first non-ionic emulsifier may have an HLB value greater than the required HLB value of the natural oil. The concept "required HLB value" is here understood, as conventional in the art, as the HLB value identified for an individual oil, necessary to give good oil-in-water emulsification. The first non-ionic emulsifier is polyoxyethylene sorbitan mono-oleate. An example of a suitable emulsifier is Tween^{®} 80.

The second non-ionic emulsifier may have an HLB value smaller than the required HLB value of the natural oil. The second non-ionic emulsifier is sorbitan mono-oleate.

An example of a suitable emulsifier is Span^{®} 80.

The formulation may comprise the first non-ionic emulsifier and the second non-ionic emulsifier in a quantitative ratio that corresponds to the required HLB value of the natural oil. The HLB values of commercially available emulsifiers are known as such from the literature or provided by their manufacturers. The required HLB value of an oil may be determined experimentally by following general procedures known in the art concerning the HLB system (ICI Americas 1984).

The formulation may comprise the first non-ionic emulsifier and the second non-ionic emulsifier in a molar ratio from 1:1 to 2:8:1.

In the formulation according to the invention the first non-ionic emulsifier and the second non-ionic emulsifier have a total molar concentration which is 80 - 150 %, preferably 90 - 140 %, more preferably 95 - 130 %, of the total molar concentration of unsaturated fatty acid(s) of the natural oil. It has been unexpectedly observed that when the total molar concentration of unsaturated fatty acid chains of the employed emulsifiers is approximately equal to the total molar concentration of unsaturated fatty acid chains of the natural oil triglycerides, the emulsifying efficiency is improved, resulting in an optically transparent, stable and homogenous microemulsion formulation in which the surface tension is in the desired range of 40 -43 mN/m.

The oil particles in the microemulsion of the present formulation may have an average hydrodynamic diameter of 100 nm or less, preferably 50 nm or less, more preferably 30 nm or less. The particle size distribution and the average hydrodynamic diameter of emulsion particles may be determined with dynamic light scattering techniques, such as by using the Zetasizer Nano ZS instrument (Malvern Instruments, Malvern, UK). The average hydrodynamic diameter may be in the range of 0.5 - 100 nm, preferably 0.8 - 50 nm, more preferably 1 - 30 nm. Due to the small particle size, the formulations may be sterile filtered by conventional filtration methods and aseptically filled into various kinds of pre-sterilized primary packages, thus avoiding possible degradation processes in the formulation or in the package materials during autoclaving at high temperature and pressure. The small particle size of the oil particles also improves the stability as well as the transparency of the formulation.

The formulation may have an optical absorbance less than 0.3 absorbance units for a 1-cm path length in a wavelength range of 400 - 700 nm, when measured from an undiluted sample of the microemulsion formulation in a quartz cuvette. In practice this means that the formulation is optically transparent and reduces the risk for blurring of vision when the formulation is administered into the eye.

The ophthalmic formulation may further comprise at least one co-emulsifier, such as glycerol. Glycerol may augment the formation of the transparent and stable microemulsion formulation. Glycerol may also provide beneficial health effects on the ocular surface.

According to one embodiment of the invention the oil particles may have a negative zeta potential, preferably from -100 mV to -0.5 mV, more preferably from -50 mV to -5 mV. Emulsion particles with negative zeta potential will repel each other, preventing flocculation and stabilizing the microemulsion. Furthermore, a negative zeta potential of the oil particles may advantageously reduce or even completely eliminate pain in the eye which is a commonly reported side effect associated with the use of emulsion formulations with a positive zeta potential. It is assumed that the oil particles with a negative zeta potential are repelled by the negatively charged eye surface epithelium, which may avoid painful sensations associated with binding to the ocular epithelium. On the contrary, the negative zeta potential of the oil particles may promote their presence in the outermost lipid layer of the tear film. In this manner, the formulation is also able effectively to counteract the adversely elevated surface tension of the tear fluid in patients with an ocular surface disease, such as dry eye.

According to one preferable embodiment the formulation is free of organic solvents and/or preservatives. As the microemulsion may be prepared without any use of organic solvents, such as chloroform, dichloromethane, tetrahydrofuran or ethanol, the occupational safety at the manufacturing site of the formulation is improved. Furthermore, as the formulation does not contain any residual amounts of organic solvents and/or preservatives, the risk for toxicity to ocular epithelial cells and allergic reactions is reduced.

The continuous water phase of the microemulsion may contain a buffering agent, such as phosphate, borate, trometamol, citrate or any of their combinations. The buffering agent is selected in view of clinical safety of the buffering agent itself when used in the eye and the desired pH range of the formulation. Typically, the pH of the formulation suitable for topical ocular administration may be in the range of 6.0 - 8.0, preferably 6.8 - 7.7. The pH of the formulation is thus within the physiologic pH range of 5.2 - 8.6 found in the tear fluid of healthy eyes, and the pH range of 3 - 8.6 tolerated by the human eye.

The formulation according to the present invention is suitable for use as a medicament. Especially the present formulation is suitable for use in the treatment of an ocular surface disorder. The ocular surface disorder may comprise impaired tear film stability, impaired tear film retention and/or tear film spreading, or tear film hyperosmolarity. The formulation according to the present invention is especially suitable use in the treatment of an ocular surface disorder selected from dry eye disease, blepharitis, Stevens-Johnson syndrome, Sjögren's syndrome, ocular cicatricial pemphigoid or graft-versus-host disease.

It is further possible to incorporate one or more active agents into the oil particles of the microemulsion of the formulation, if desired.

The formulation may be administered topically in form of eye drops, eye gel, eye ointment, eye wash, eye mist, eyelid wipe or eye spray. The formulation is suitable for topical administration to the eye or on the eyelid. More generally the formulation according to the present invention is suitable for administration on a mucous membrane, e.g. on nasal mucous membrane. Especially the formulation present invention is suitable for use in the treatment of an ocular surface disorder, where the formulation may be administered topically in form of eye drops, eye gel, eye ointment, eye wash, eye mist, or eye spray.

The optically clear and thermodynamically stable formulations according to the present invention can be filled, if desired, in transparent or translucent primary packages and used without the need for shaking, thus increasing the commercial appeal. This means that the present formulation is optically transparent, stable and homogenous, requiring no restoration of the homogeneity of the formulation prior to the application.

### LIST OF FIGURES

Figures 1A - 1F show clinical efficacy of SIME02A, denoted as SIME, and HA control eye drops for signs of dry eye.
Figure 1A: Ocular protection index (OPI); mean ± SD (ITT population, n=26 per group). *Dash line* marks OPI=1.
Figure 1B: Tear osmolarity in patients with baseline tear hyperosmolarity (≥308 mOsm/kg, *dash line*); mean ± SD (ITT population, n=8 for SIME, n=14 for HA).
Figure 1C: Corneal staining; mean ± SD of the mean values of eyes (Oxford scale 0-5) (PP population, n=24 for SIME; n=25 for HA).
Figure 1D: Nasal conjunctival staining; mean ± SD of the mean values of eyes (Oxford scale 0-5) (PP population, n=24 for SIME; n=25 for HA).
Figure 1E: Conjunctival redness; mean ± SD of the mean values of eyes (IER scale 0-4) (safety/ITT population, n=26).
Figure 1F: Lid redness; mean ± SD of the mean values of eyes (IER scale 0-4) (safety/ITT population, n=26).
Figures 2A - 2D show box-whisker plot of ocular surface disease index (OSDI). The mean is marked with a diamond. Boxes represent interquartile ranges separated by the median; whiskers indicate the minimum and maximum values (ITT population, n=26). ***, p<0.0001.

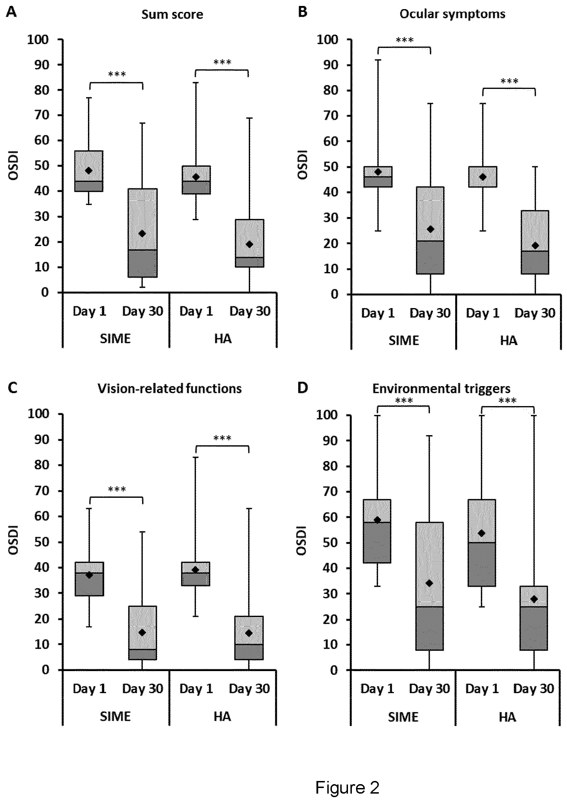

Figure 2A: OSDI sum score.
Figures 2B - 2D: Subscale scores of OSDI.

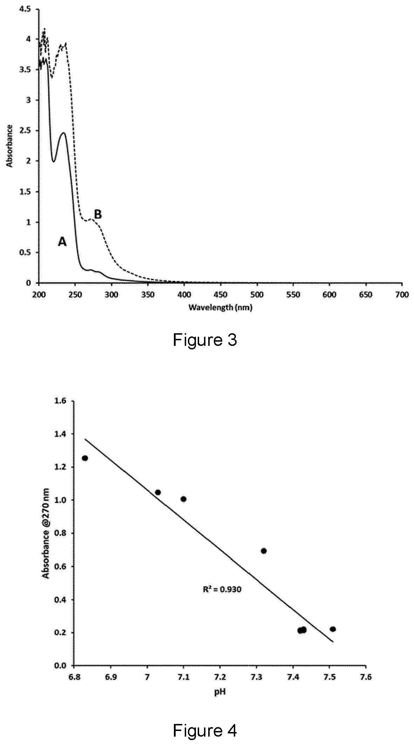

Figure 3 shows UV-VIS absorption spectrum of microemulsion SIME04 with sacha inchi seed oil. No significant absorption in the visible wavelength range 400-700 nm is observed in emulsion solutions stored (A) at room temperature (about 20-25 °C) or (B) at about 45 °C for 3 months.
Figure 4 shows correlation of pH and UV absorption (at 270 nm) of microemulsion SIME04.

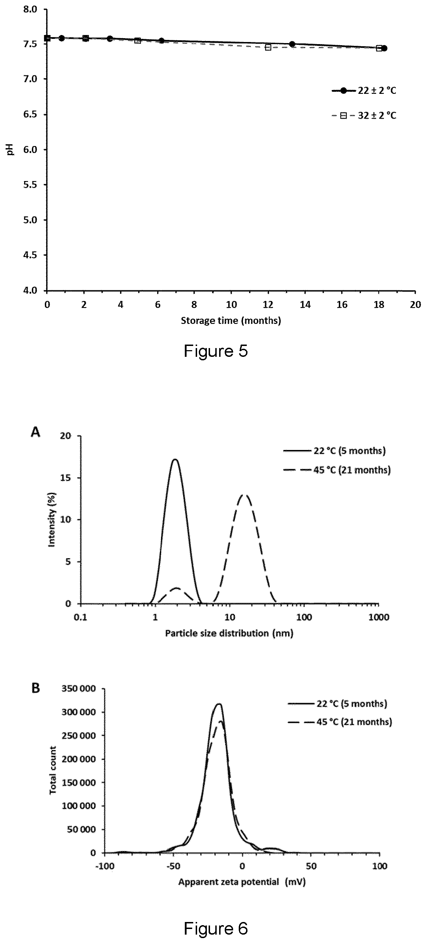

Figure 5 shows stability analysis of microemulsion SIME02A; mean ± SD, n=3. SD bars may not be visible due to their small values.
Figure 6 shows hydrodynamic diameter (A) and zeta potential (B) distribution of microemulsion SIME02A stored at room temperature (22 °C) and at 45 °C.

### EXAMPLES

Some embodiments of the invention are described in the following non-limiting examples.

### Example 1

A series of microemulsion formulations was prepared according to Table 1. All chemicals are generally available from various commercial suppliers. The formulation comprised sacha inchi seed oil (natural oil), Tween^{®} 80 (the first non-ionic emulsifier) and Span^{®} 80 (the second non-ionic emulsifier), and glycerol (co-emulsifier). The needed concentration of the first and second non-ionic emulsifier to emulsify a certain percentage of oil was determined according to general instructions concerning the HLB system (ICI Americas 1984) by using spectral absorption in the UV/visible wavelength range as an endpoint. The HLB values for Span^{®} 80 and Tween^{®} 80 are known from the literature. The most optimal concentrations were those producing the least absorption. Fine-tuning of the concentrations by use of mathematical fitting procedures for the absorption data resulted in the required HLB value for the oil. Next, the required minimum total amount of both emulsifiers per the amount of oil in the obtained HLB ratio were subsequently determined. As the last step, the required minimum concentration of the co-emulsifier was determined as well. After these adjustments, the relative concentrations of oil, emulsifiers and co-emulsifier are known, and the microemulsion composition has the lowest possible absorbance (maximal transmittance) and visual transparency. At each step explained, the lipid component was mixed by using a conventional magnetic stirrer with heating to above about 50 °C. The aqueous buffered solution containing sodium hyaluronate, KCl and trehalose was gradually added until the desired oil concentration was reached. Without any further adjustments, the surface tension was measured using Krüss K6 force tensiometer by the ring method (Krüss GmbH, Hamburg, Germany) at room temperature. A control solution CS01 was prepared without a lipid component for reference.

The results presented in Table 1 suggest that the lipid component comprising natural oil contributes to lowering of the surface tension. Even up to 5-fold changes (0.1% → 0.5%) in the amount of the natural oil do not shift the surface tension outside the 40 - 43 mN/m range. It can also be seen in Table 1 that the drop volume obtained from a conventional eye drop bottle is affected by surface tension; the most preferable volume that the human eye can hold is about 30 µl which can be obtained with microemulsion formulations comprising the lipid component. The eye drop volume must be strictly distinguished from the phenomenon of droplet or particle size of the emulsion.

### Example 2

A microemulsion formulation SIME02A was prepared. The composition of SIME02A corresponds to the composition of SIME02 of Table 1, except the amount of sodium hyaluronate (HA) was 0.2 weight-%. A control formulation comprised 0.2% sodium hyaluronate (HA) in isotonic phosphate-buffered saline without natural oil. The formulations were visually similar (clear, homogenous and colourless), sterile filtered and aseptically filled into identical eye drop bottles designed for preservative-free administration, allowing complete masking of the treatments from the patients and the investigator. Fifty-two adult patients with moderate or severe dry eye, allocated into randomized SIME02A or control groups (n=26 for both), self-administered the corresponding eye drops on both eyes three times a day for about 30 days.

On Day 30, with no morning dose taken, clinical assessments were performed. All study assessments at baseline and on Day 30 were performed in an examination room monitored for temperature and relative humidity (RH). Safety and ocular tolerability, evaluated as the primary objective, included best corrected visual acuity (ETDRS charts 1 & 2, 2000 series), conjunctival (bulbar) and lid redness (IER grading for both; 0=not existing, 1=very slight, 2=slight, 3=moderate, 4=severe), intraocular pressure (IOP; Goldmann tonometer), digital photography of the anterior eye, and visual assessment of ocular reactions. Ocular biomarkers used as primary efficacy endpoints included tear osmolarity (I-PEN^{®} Tear Osmolarity System, I-MED Pharma, Dollard-des-Ormeaux, QC, Canada) and fluorescein TBUT. For secondary efficacy objectives, blink rate and corneal and conjunctival staining (Oxford grading 0-5 for both) were assessed; blink rate (min⁻¹) was converted to interblink interval (IBI; s) to calculate the ocular protection index (OPI=TBUT/IBI). The ocular surface disease index (OSDI) was used for both safety and efficacy. Adverse events (AEs) were collected throughout the 30-day test period.

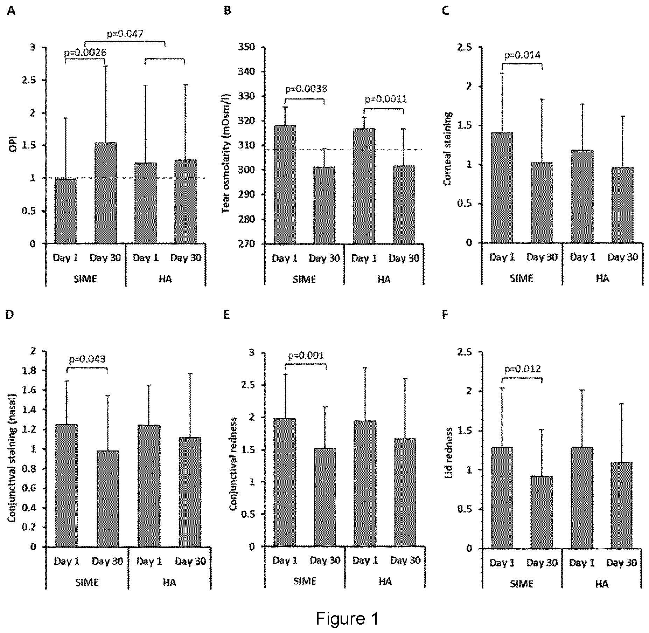

A total of 49 subjects completed the 30-day test period per protocol (PP); safety data was obtained from all 52 randomized intent-to-treat (ITT) subjects. Both formulations were well tolerated without treatment-related AEs. TBUT increased significantly by about 50% with SIME02A and non-significantly by 25% with control HA, whereas blink rates did not change considerably (Table 2). OPI improved significantly by 57% to about 1.5 OPI units with SIME02A (p=0.0026, ANOVA) and by 4% with HA; treatment difference in changes from baseline was statistically significant for ITT population (p=0.047) and marginally significant for PP population (p=0.071) in favour of SIME02A (Fig. 1A, Table 2).

In both SIME02A group and the control group, mean tear osmolarity was initially <308 mOsm/l and showed a small decrease by Day 30 (Table 2). In hyperosmolar ITT and PP datasets, tear osmolarities reduced significantly to about 300 mOsm/l with both treatments (Fig. 1B, Table 2).

The effect of SIME02A formulation on all staining scores assessed after 30 days was greater than that of control formulation HA, although without statistical difference between treatments (Table 2). Mean ocular staining scores were of absent-to-mild intensity at baseline (0-2 score units on Oxford scale 0-5). Corneal staining decreased significantly with SIME02A in PP dataset (-27%, p=0.014, Wilcoxon signed rank test) and marginally significantly in ITT subjects (-20%, p=0.077) (Fig. 1C, Table 2). Similarly, SIME02A reduced conjunctival staining with statistical significance in the nasal conjunctiva of PP subjects (-22%, p=0.043) and with marginal significance in ITT subjects (-19%, p=0.059) (Fig. 1D, Table 2).

Significantly reduced mean conjunctival (-23%, p=0.001) and lid redness (-29%, p=0.012) scores were observed with SIME02A in the safety/ITT dataset, while changes in the control group remained milder (ca. -15%) and non-significant (Fig. 1E and 1F, Table 2).

Finally, the mean OSDI sum score decreased with both SIME02A formulation (-51%) and control formulation HA (-58%) (ITT dataset; p<0.0001 for PP and ITT populations), most patients reporting mild or non-existent residual symptoms on Day 30 (medians 17 and 14, respectively) (Fig. 2, Table 2). Similarly, a highly significant reduction was seen in all three OSDI subscales (p<0.0001) (Fig. 2B-D). Of interest, the median of "vision-related functions" subscale decreased from 38 score units in both groups to ≤10 units (Fig. 2C), suggesting that the majority of subjects were effectively without vision-related functional symptoms after 30 days.

The results of the study suggest that SIME02A formulation was able to improve each of the three etiologic factors for dry eye, comprising (1) tear film instability, (2) tear hyperosmolarity and (3) cellular damage and inflammation, as categorized in Table 2, while the non-lipid control treatment was able to correct hyperosmolarity only. Because the control formulation HA contained only sodium hyaluronate, it can be deduced that the lipid component comprising natural oil of SIME02A was responsible for the clinical treatment effect. The surface tension measured in a solution corresponding to the control formulation HA and additionally supplemented with 2% trehalose is 73.7 mN/m; thus, as the surface tension of the control solution CS01 is 68 mN/m (Table 1), the surface tension of the control formulation HA used in the clinical trial must be in the range 68 - 73.7 mN/m, i.e. in a close proximity to that of pure water (Table 1). The shown improvements in the etiologic factors were accompanied by resolution of dry eye symptoms (Fig. 2, Table 2), confirming both clinical safety and efficacy of SIME02A.

### Example 3

A microemulsion formulation SIME04, as described in Table 3 was prepared, sterile filtered and aseptically filled into bag-in-bottle-type eye spray containers designed for preservative-free administration. The bottles were subjected to stability testing at temperatures 22 ± 2 °C (sample A) and 45 ± 2 °C (sample B) for 3 months. At the end of testing period, sample B was visually clearly more turbid but still perfectly homogenous. Absorption spectra measured over 200 to 700 nm (Fig. 3) for samples A and B after 3-month storage show that very small changes take place in the visible wavelength range 400 - 700 nm, whereas larger changes are seen in the UV range at below 350 nm at 45 ± 2 °C. The absorbance of sample A after 3-month storage corresponded to the initial absorbance at time of manufacture (not shown in Fig. 3), demonstrating no significant changes at 22 ± 2 °C.

In a further step, samples of microemulsion formulations SIME04 were stored for various time periods in different conditions and analysed for pH and absorbance at 270 nm. The wavelength 270 nm was selected based on a large absorbance change at that wavelength, as shown in Fig. 3. The results are shown in Fig. 4 where both parameters are plotted in the same graph. It can be seen that there is a strong correlation between the pH of the solution and its UV absorbance. This observation reveals that pH can be used as a convenient proxy parameter for stability analyses of microemulsions.

### Example 4

Three separate batches of a microemulsion formulation SIME02A as in Example 2 were prepared, sterile filtered and aseptically filled into gamma-sterilized semitransparent eye drop bottles designed for preservative-free administration. Sample bottles from these batches were subjected to stability testing at temperatures 22 ± 2 °C and 32 ± 2 °C. pH was used as a proxy parameter for changes in the microemulsion formulation according to Example 3. The results presented in Fig. 5 indicate that microemulsion formulation SIME02A was stable for the studied time period of up to 18 months at both temperature conditions; pH did not significantly decrease, corresponding to visual transparency which was similar to the initial microemulsion with no visible turbidity, particles, separation of phases, or colour changes.

### Example 5

Microemulsion formulation SIME02A as in Example 2 was prepared. The hydrodynamic size distribution of the microemulsion particles were measured with dynamic light scattering (Zetasizer Nano ZS instrument, Malvern Instruments, Malvern, UK) using 173° back-scattered light signal at 25 °C. The hydrodynamic diameter of the particles was calculated using the intensity-weighted Stokes-Einstein equation. Zeta potential measurements were performed with electrophoretic light scattering (Zetasizer Nano ZS) at 25 °C.

The obtained particle size distribution presented in Fig. 6 shows that the average hydrodynamic diameter was 1.4 ± 0.1 nm in samples stored at 22 °C, indicating a transparent and monodisperse microemulsion with very small particles. After storage for 21 months at 45 °C, the majority of emulsion particles were about ten times larger, while a small part retained their original size (Fig. 6A), still indicating a transparent microemulsion. The average zeta potential of the particles in the same samples was -19 ± 1 mV at 22 °C and -18 ± 2 at 45 °C, demonstrating practically unchanged zeta potential (Fig. 6B).

It is apparent to a person skilled in the art that the invention is not limited exclusively to the examples described above, but that the invention can vary within the scope of the claims presented below.

**Table 1 Composition and properties of microemulsion formulations (SIME), control solution (CS01) and water.**

| **Reagent** | **SIME01** | **SIME02** | **SIME03** | **CS01** | **Water** |
|---|---|---|---|---|---|
| Trometamol | 0.29% | 0.29% | 0.29% | 0.29% | - |
| Citric acid monohydrate | 0.14% | 0.14% | 0.14% | 0.14% | - |
| Sodium hyaluronate | 0.20% | 0.25% | 0.25% | 0.25% | - |
| Trehalose dihydrate | 2.0% | 2.0% | 2.0% | 2.0% | - |
| Potassium chloride | 0.17% | 0.17% | 0.17% | 0.17% | - |
| Sacha inchi seed oil | 0.5% | 0.1% | 0.3% | - | - |
| Tween^{®} 80 | 1.51% | 0.30% | 0.91% | - | - |
| Span^{®} 80 | 0.34% | 0.068% | 0.21% | - | - |
| Glycerol | 0.88% | 0.88% | 0.88% | 0.88% | - |
| Purified water | ad 100% | ad 100% | ad 100% | ad 100% | ad 100% |

| **Measurements** | | | | | |
|---|---|---|---|---|---|
| pH | 7.57 | 7.54 | 7.55 | nd¹ | nd¹ |
| Osmolality (mOsm/kg) | 246 | 242 | 245 | 236 | 0 |
| Drop size (µl) ² | 36.7 | 30.0 | 26.7 | 40.0 | nd¹ |
| Surface tension (mN/m) | 41.5 | 42.6 | 42.6 | 68.0 | 70.0 |

| | | | | | |
|---|---|---|---|---|---|
| ¹nd, not determined; determined by using identical eye dropper bottles for each solution | | | | | |

**Table 3. Composition of microemulsion SIME04**

| **Reagent** | **SIME04** |
|---|---|
| Trometamol | 0.29% |
| Citric acid monohydrate | 0.14% |
| Sodium hyaluronate | 0.02% |
| Sodium chloride | 0.19% |
| Potassium chloride | 0.04% |
| Sacha inchi seed oil | 0.5% |
| Tween^{®} 80 | 1.52% |
| Span^{®} 80 | 0.33% |
| Glycerol | 0.85% |
| Purified water | ad 100% |

| **Measurements** | |
|---|---|
| pH | 7.51 |
| Osmolality (mOsm/kg) | 207 |

**Table 2 Summary of results categorized by dry eye etiology, Example 2**

| **Etiologic factor and symptoms** | | **Measured variable (unit)** | **Study population** | **Mean ± SD change from baseline on Day 30** | | |
|---|---|---|---|---|---|---|
| | | | | **SIME02** ITT, n=26; | **HA** ITT, n=26; | **Effect size; 95% Cl¹** |
| | | | | PP, n=24 | PP, n=25 | |
| **1. Tear film instability** | | TBUT (s) | ITT | 1.71±3.42 (p=0.0025)* | 0.87±1.81 (p=0.11) | 0.85; -0.68 to 2.37 (p=0.27) |
| | | | PP | 1.65±3.48 (p=0.0055)* | 0.88±1.84 (p=0.12) | 0.77; -0.83 to 2.36 (p=0.34) |
| | | Blink rate (min⁻¹) | ITT | 0.02± 8.72 (p=0.99) | -2.15±10.2 (p=0.25) | 2.17; -3.10 to 7.45 (p=0. 41) |
| | | | PP | -0.19 ± 9.03 (p=0.93) | -1.96±10.3 (p=0.32) | 1.77; -3.81 to 7.36 (p=0.53) |
| | | OPI (ratio) | ITT | 0.56±1.08 (p=0.0026)* | 0.05±0.68 (p=0.78) | 0.51; 0.0073 to 1.01 (p=0.047) * |
| | | | PP | 0.53±1.11 (p=0.0068)* | 0.05±0.69 (p=0.80) | 0.49; -0.043 to 1.02 (p=0.071) |
| **2. Tear film hyperosmolarity** | | Tear osmolarity, all (mOsm/l) | ITT | -4.75±16.2 (p=0.21) | -1.77±21.5 (p=0.64) | -2.98; -13.6 to 7.64 (p=0.58) |
| | | | PP | -4.73±14.6 (p=0.22) | -2.36±21.8 (p=0.53) | -2.37; -13.1 to 8.32 (p=0.65) |
| | | Tear osmolarity, ≥308 (mOsm/l) | ITT | -17.1±10.4 (p=0.0038)* | -15.1±16.6 (p=0.0011) * | -1.99; -15.6 to 11.6 (p=0.76) |
| | | | PP | -14.6±8.5 (p=0.015)* | -15.1±16.6 (p=0.0010) * | 0.43; -13.7 to 14.5 (p=0.95) |
| **3A. Ocular surface damage** | | Corneal staining (Oxford scale 0-5) | ITT | -0.27±0.78 (p=0.077) | -0.21±0.51 (p=0.070) | -0.06 (p=0.77) |
| | | | PP | -0.38±0.70 (p=0.014)* | -0.22±0.52 (p=0.070) | -0.16 (p=0.45) |
| | | Conjunctival staining, temporal (Oxford scale 0-5) | ITT | -0.17±0.51 (p=0.13) | -0.02±0.79 (p=0.90) | -0.15 (p=0.53) |
| | | | PP | -0.19±0.51 (p=0.12) | -0.02±0.81 (p=0.90) | -0.17 (p=0.52) |
| | | Conjunctival staining, nasal (Oxford scale 0-5) | ITT | -0.23±0.60 (p=0.059) | -0.12±0.65 (p=0.44) | -0.11 (p=0.73) |
| | | | PP | -0.27±0.61 (p=0.043)* | -0.12±0.67 (p=0.44) | -0.15 (p=0.59) |
| **3B. Ocular surface inflammation** | | Conjunctival redness (IER grading 0-4) | Safety/ITT | -0.46±0.65 (p=0.001)* | -0.27±0.65 (p=0.065) | -0.19 (p=0.36) |
| | | Lid redness (IER grading 0-4) | Safety/ITT | -0.37±0.67 (p=0.012)* | -0.19±0.55 (p=0.13) | -0.18 (p=0.30) |
| | Sum of planned primary and secondary end points with significant improvement from baseline² | | Safety/ITT | 4 | 1 | 1 |
| | | | PP | 4 | 1 | 0 |
| **Symptoms** | | OSDI sum score (scale 0-100) | ITT | -24.6±16.2 (p<0.0001) * | -26.5±14.6 (p<0.0001) * | 1.9 (p=0.66) |
| | | | PP | -25.0±16.5 (p<0.0001) * | -27.2±14.5 (p<0.0001) * | 2.2 (p=0.62) |

| | | | | | | |
|---|---|---|---|---|---|---|
| ¹Estimate of SIME02 vs. HA difference, 95% confidence interval for continuous variables, and between-group significance of change ²Excluding *ad hoc* analyses *, Statistically significant difference (p<0.05) | | | | | | |

## Claims

1. An ophthalmic formulation in form of an oil-in-water microemulsion comprising oil particles dispersed in a continuous water phase, the formulation comprising
- 0.1 - 2.0 weight-% of a natural oil, which is sacha inchi seed oil, and
- at least a first non-ionic emulsifier, which is polyoxyethylene sorbitan mono-oleate and a second non-ionic emulsifier, which is sorbitan mono-oleate
wherein the first non-ionic emulsifier and the second non-ionic emulsifier have a total molar concentration which is 80 - 150 % of the total molar concentration of unsaturated fatty acid(s) of the natural oil,
wherein the formulation has a surface tension in a range of 40 - 43 mN/m at an emulsion-air interface at a temperature of 15 - 30 °C.

2. The formulation according to claim 1, **characterised in that** the first non-ionic emulsifier and the second non-ionic emulsifier have a total molar concentration which is 90 - 140 %, preferably 95 - 130 %, of the total molar concentration of unsaturated fatty acid(s) of the natural oil.

3. The formulation according to claim 1 or 2, **characterised in** the that the oil particles have an average hydrodynamic diameter of 100 nm or less, preferably 50 nm or less, more preferably 30 nm or less.

4. The formulation according to claim 1, 2 or 3, **characterised in that** the oil particles have a negative zeta potential, preferably from -100 mV to -0.5 mV, more preferably from -50 mV to -5 mV.

5. The formulation according to any of preceding claims 1 - 4, **characterised in that** the formulation has an optical absorbance less than 0.3 absorbance units for a 1-cm path length in a wavelength range of 400 - 700 nm.

6. The formulation according to any of preceding claims 1 - 5, **characterised in that** the formulation is free of organic solvents and/or preservatives.

7. A formulation according to any of claims 1 - 6 for use as a medicament.

8. A formulation according to any of claims 1 - 6 for use in a treatment of an ocular surface disorder.

9. The formulation according to any of claims 1 - 6 for use in the treatment of an ocular surface disorder according to claim 8, **characterised in that** the ocular surface disorder comprises impaired tear film stability, impaired tear film retention and/or tear film spreading, or tear film hyperosmolarity.

10. The formulation according to any of claims 1 - 6 for use in the treatment of an ocular surface disorder according to claim 8 or 9, **characterised in that** the ocular surface disorder is selected from dry eye disease, blepharitis, Stevens-Johnson syndrome, Sjögren's syndrome, ocular cicatricial pemphigoid or graft-versus-host disease.

11. The formulation according to any of claims 1 - 6, for use in the treatment of an ocular surface disorder according to claim 8, 9 or 10, **characterised in that** the formulation is administered topically in form of eye drops, eye gel, eye ointment, eye wash, eye mist, or eye spray.

## Patentansprüche

1. Ophthalmische Formulierung in Form einer Öl-in-Wasser-Mikroemulsion, umfassend in einer kontinuierlichen Wasserphase dispergierte Ölpartikel, wobei die Formulierung Folgendes umfasst
- 0,1- 2,0 Gew.-% eines natürlichen Öls, das Sacha-Inchi-Samenöl ist, und
- mindestens einen ersten nichtionischen Emulgator, der Polyoxyethylensorbitanmonooleat ist, und einen zweiten nichtionischen Emulgator, der Sorbitanmonooleat ist, wobei der erste nichtionische Emulgator und der zweite nichtionische Emulgator eine molare Gesamtkonzentration aufweisen, die 80-150 % der molaren Gesamtkonzentration an ungesättigter(n) Fettsäure(n) des natürlichen Öls beträgt,
wobei die Formulierung eine Oberflächenspannung in einem Bereich von 40-43 mN/m an einer Emulsions-Luft-Grenzfläche bei einer Temperatur von 15-30°C aufweist.

2. Formulierung nach Anspruch 1, **dadurch gekennzeichnet, dass** der erste nichtionische Emulgator und der zweite nichtionische Emulgator eine molare Gesamtkonzentration aufweisen, die 90-140 %, vorzugsweise 95-130 % der gesamten molaren Konzentration an ungesättigter(n) Fettsäure(n) des natürlichen Öls ausmacht.

3. Formulierung nach Anspruch 1 oder 2, **dadurch gekennzeichnet, dass** die Ölpartikel einen durchschnittlichen hydrodynamischen Durchmesser von 100 nm oder weniger, vorzugsweise 50 nm oder weniger, besonders bevorzugt 30 nm oder weniger aufweisen.

4. Formulierung nach Anspruch 1, 2 oder 3, **dadurch gekennzeichnet, dass** die Ölpartikel ein negatives Zeta-Potential vorzugsweise von -100 mV bis -0,5 mV, mehr bevorzugt von -50 mV bis -5 mV aufweisen.

5. Formulierung nach einem der vorhergehenden Ansprüche 1 bis 4, **dadurch gekennzeichnet, dass** die Formulierung eine optische Extinktion von weniger als 0,3 Extinktionseinheiten für eine Weglänge von 1 cm in einem Wellenlängenbereich von 400-700 nm aufweist.

6. Formulierung nach einem der vorhergehenden Ansprüche 1 bis 5, **dadurch gekennzeichnet, dass** die Formulierung frei von organischen Lösungsmitteln und/oder Konservierungsmitteln ist.

7. Formulierung nach einem der Ansprüche 1 bis 6 zur Verwendung als Medikament.

8. Formulierung nach einem der Ansprüche 1 bis 6 zur Verwendung bei der Behandlung einer Erkrankung der Augenoberfläche.

9. Formulierung nach einem der Ansprüche 1 bis 6 zur Verwendung bei der Behandlung einer Augenoberflächenstörung nach Anspruch 8, **dadurch gekennzeichnet, dass** die Störung der Augenoberfläche eine beeinträchtigte Tränenfilmstabilität, eine beeinträchtigte Tränenfilmretention und/oder Tränenfilmausbreitung oder eine Tränenfilmhyperosmolarität umfasst.

10. Formulierung nach einem der Ansprüche 1 bis 6 zur Verwendung bei der Behandlung einer Augenoberflächenstörung nach Anspruch 8 oder 9, **dadurch gekennzeichnet, dass** die Erkrankung der Augenoberfläche ausgewählt ist aus Augentrockenheit, Blepharitis, Stevens-Johnson-Syndrom, Sjögren-Syndrom, vernarbendem Augenpemphigoid oder Graft-versus-Host-Erkrankung.

11. Formulierung nach einem der Ansprüche 1 bis 6 zur Verwendung bei der Behandlung einer Augenoberflächenstörung nach Anspruch 8, 9 oder 10, **dadurch gekennzeichnet, dass** die Formulierung topisch in Form von Augentropfen, Augengel, Augensalbe, Augenspülung, Augennebel oder Augenspray verabreicht wird.

## Revendications

1. Formulation ophtalmique sous la forme d'une microémulsion huile-dans-eau comprenant des particules d'huile dispersées dans une phase aqueuse continue, la formulation comprenant
- 0,1 à 2,0 % en poids d'une huile naturelle, qui est l'huile de graines de sacha inchi, et
- au moins un premier émulsifiant non ionique qui est le monooléate de polyoxyéthylène sorbitane et un second émulsifiant non ionique qui est le monooléate de sorbitane,
dans laquelle le premier émulsifiant non ionique et le second émulsifiant non ionique ont une concentration molaire totale qui est de 80 à 150 % de la concentration molaire totale d'acide(s) gras insaturé(s) de l'huile naturelle,
dans laquelle la formulation a une tension superficielle dans une plage de 40 à 43 mN/m à une interface émulsionair à une température de 15 à 30 °C.

2. Formulation selon la revendication 1, **caractérisée en ce que** le premier émulsifiant non ionique et le second émulsifiant non ionique ont une concentration molaire totale qui est de 90 à 140 %, de préférence de 95 à 130 %, de la concentration molaire totale d'acide(s) gras insaturé(s) de l'huile naturelle.

3. Formulation selon la revendication 1 ou 2, **caractérisée en ce que** les particules d'huile ont un diamètre hydrodynamique moyen de 100 nm ou moins, de préférence de 50 nm ou moins, de manière davantage préférée de 30 nm ou moins.

4. Formulation selon la revendication 1, 2 ou 3, **caractérisée en ce que** les particules d'huile ont un potentiel zêta négatif, de préférence de -100 mV à - 0,5 mV, de manière davantage préférée de -50 mV à -5 mV.

5. Formulation selon l'une quelconque des revendications précédentes 1 à 4, **caractérisée en ce que** la formulation a une absorbance optique inférieure à 0,3 unité d'absorbance pour une longueur de trajet de 1 cm dans une plage de longueurs d'onde de 400 à 700 nm.

6. Formulation selon l'une quelconque des revendications précédentes 1 à 5, **caractérisée en ce que** la formulation est exempte de solvants organiques et/ou de conservateurs.

7. Formulation selon l'une quelconque des revendications 1 à 6 destinée à être utilisée comme médicament.

8. Formulation selon l'une quelconque des revendications 1 à 6 destinée à être utilisée dans un traitement d'un trouble de la surface oculaire.

9. Formulation selon l'une quelconque des revendications 1 à 6 destinée à être utilisée dans le traitement d'un trouble de la surface oculaire selon la revendication 8, **caractérisée en ce que** le trouble de la surface oculaire comprend une stabilité altérée du film lacrymal, une rétention altérée du film lacrymal et/ou un étalement du film lacrymal, ou une hyperosmolarité du film lacrymal.

10. Formulation selon l'une quelconque des revendications 1 à 6, destinée à être utilisée dans le traitement d'un trouble de la surface oculaire selon la revendication 8 ou 9, **caractérisée en ce que** le trouble de la surface oculaire est choisi parmi la sécheresse oculaire, la blépharite, le syndrome de Stevens-Johnson, le syndrome de Sjögren, la pemphigoïde oculaire cicatricielle ou la maladie du greffon contre l'hôte.

11. Formulation selon l'une quelconque des revendications 1 à 6, destinée à être utilisée dans le traitement d'un trouble de la surface oculaire selon la revendication 8, 9 ou 10, **caractérisée en ce que** la formulation est administrée par voie topique sous forme de gouttes oculaires, de gel oculaire, de pommade oculaire, de collyre, de brouillard oculaire ou de vaporisateur oculaire.
